# EUROPEAN PATENT APPLICATION

(11) **EP 3 329 961 A1**
(43) Date of publication of application: **06.06.2018**
(21) Application number: 16833272.4
(22) Date of filing: 29.07.2016
(51) Int. Cl.: A61N 1/05, A61M 37/00, B81B 7/02, B81B 1/00, B81B 3/00, B81C 3/00, B81C 1/00

(54) **IMPLANTABLE LEAD FOR STIMULATING COMPLEX NERVES AND MANUFACTURING METHOD THEREFOR**

(30) Priority: 31.07.2015 KR 20150109075
(71) Applicant: Osong Medical Innovation Foundation, Cheongju-si, Chungcheongbuk-do 28160 (KR)
(72) Inventor: MOON, Jin-hee, Sejong 30031 (KR); SONG, In-ho, Seoul 02799 (KR); KIM, Jin-won, Seoul 07980 (KR); LEE, Seung-a, Seoul 04354 (KR); JUNG, Ha-chul, Cheongju-si Chungcheongbuk-do 28427 (KR); AHN, Jin-woo, Cheongju-si Chungcheongbuk-do 28160 (KR); LEE, Sang-hun, Cheongju-si Chungcheongbuk-do 28160 (KR); KIM, A-hee, Cheongju-si Chungcheongbuk-do 28160 (KR)
(74) Representative: Gaunt, Thomas Derrick
(86) International application number: PCT/KR2016/008364
(87) International publication number: WO 2017/023036

(57) **Abstract**

In an implantable hybrid lead and a method of manufacturing the implantable hybrid lead, the implantable hybrid lead includes a conduit, a line electrode and a plurality of electrode terminals. The conduit has a fine channel through which a medicine is injected. The line electrode is inserted to and is combined with an outside of the conduit, and applies electrical simulation to a selected portion of a living body. A plurality of electrode terminals is disposed at an end of the conduit by a predetermined distance.

## Description

### BACKGROUND

### 1. Field of Disclosure

The present disclosure of invention relates to an implantable hybrid lead and a method of manufacturing the implantable hybrid lead, and more specifically the present disclosure of invention relates to an implantable hybrid lead and a method of manufacturing the implantable hybrid lead, in which a thin film electrode (or thin film multi electrodes array) manufactured by micro-electromechanical manufacturing system and medicinal injecting micro channel are combined together, such that medicinal injecting and electrical stimulating are performed at the same time using a single lead.

### 2. Description of Related Technology

Recently, a lead capable of medicinal injecting and electrical stimulating at the same time and capable of being inserted and implanted into human body, may be widely used in various medical devices, such as Pacemaker, Implantable Cardioverter Defibrillator (ICD), Deep Brain Stimulator (DBS), Spinal Cord Stimulation System reducing a pain, Brain Machine Interface (BMI) controlling a robot artificial arm or leg by neural transmission, and so on. Hereinafter, a combined lead may be defined as a lead in which a thin film electrode or thin film multi electrodes array and a medicinal injecting flexible channel are combined together.

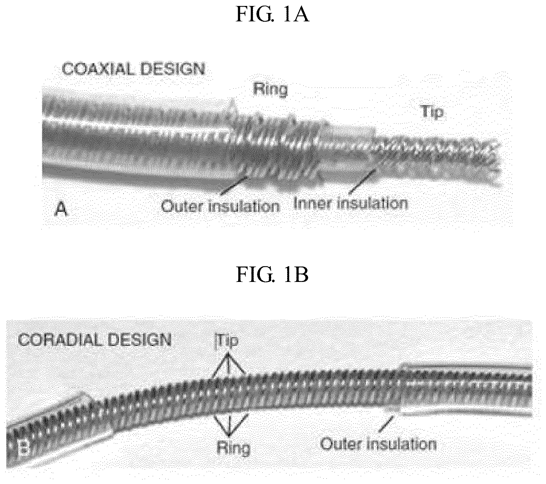

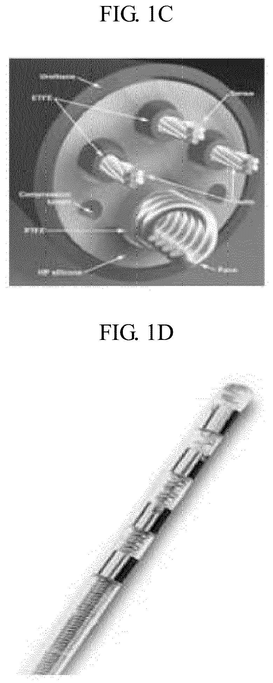

Conventionally, in the lead of an implanting medical device, as illustrated in FIGS. 1A to FIGS. 1D, platinum (Pt) or platinum-iridium (Ir) alloy wire is spirally inserted into a biocompatible and flexible medical polyurethane (PU) channel, and then the wire is electrically connected to an electrode exposed to outside of a tube disposed at an end of the lead.

However, in the above mentioned type of electrode, a thickness is increased as the number of wires increases, when the number of channels of the electrodes increases. Thus, the maximum number of the channels is limited, and an additional medicinal injecting syringe is necessary for the medicinal injecting and electrical stimulating at the same time.

Generally, in nerve disease, the medicinal injecting is initially performed, and then the electrical stimulating lead is implanted to treat the nerve disease when the medicinal injecting is not effective any more. However, at the initial term of the nerve disease, the nerve disease may be effective treated and delayed when the medicinal injecting and the electrical stimulating are performed at the same time. Thus, an implantable device in which the medicinal injecting and the electrical stimulating are performed at the same time is necessary to be developed.

Related prior arts are Korean laid-open patent application No. 10-2012-0032521 and Korean Patent No. 10-1097511.

### SUMMARY

The present invention is developed to solve the above-mentioned problems of the related arts. The present invention provides an implantable hybrid lead, in which a thin film electrode (or thin film multi electrodes array) manufactured by micro-electromechanical manufacturing system and medicinal injecting micro channel are combined together, such that medicinal injecting and electrical stimulating are performed at the same time using a single lead. In the present invention, the implantable hybrid lead may be inserted in a brain, a spinal cord, a neural tissue, a muscle or a muscle tissue, a heart, a cardiovascular and so on, and may inject the medicine and stimulate electrically at the same time.

In addition, the present invention provides a method of manufacturing the implantable hybrid lead.

According to an example embodiment, the implantable hybrid lead includes a conduit, a line electrode and a plurality of electrode terminals. The conduit has a fine channel through which a medicine is injected. The line electrode is inserted to and is combined with an outside of the conduit, and applies electrical simulation to a selected portion of a living body. A plurality of electrode terminals is disposed at an end of the conduit by a predetermined distance.

In an example, the line electrode may be a thin film electrode or thin film multi electrodes array.

In an example, the line electrode is coiled around an outer surface of the conduit to be connected with the conduit, by a predetermined distance.

According to an example embodiment, in a method of manufacturing an implantable hybrid lead, a thin film electrode is manufactured by MEMS. A conduit is manufactured using a liquid biocompatible polymer. The conduit has a fine channel through which a medicine is injected. The thin film electrode is inserted and combined with an outside of the conduit to be packaged.

According to an example embodiment, in a method of manufacturing an implantable hybrid lead, thin film multi electrodes array is manufactured by MEMS. A conduit is manufactured using a liquid biocompatible polymer. The conduit has a fine channel through which a medicine is injected. The thin film multi electrodes array is inserted and combined with an outside of the conduit to be packaged.

In an example, the method may further include inserting the conduit having the fine channel to which the thin film electrode or the thin film multi electrodes array is attached, into a mold, and injecting the liquid biocompatible polymer for molding.

In an example, the thin film electrode or the thin film multi electrodes array may be coiled around an outer surface of the conduit to be connected with the conduit, by a predetermined distance.

According to the present example embodiments, the conduit having the fine channel through which the medicine is injected, and the thin film electrode manufactured by MEMS, are combined to be packaged, and thus an implantable hybrid lead may be manufactured. Thus, a vital sign may be measured on various kinds of bio tissues and neural tissues, the electrical stimulating and the medicinal injecting may be performed at the same time.

In addition, a fatigue or a damage due to repetitive bending stimulation may be prevented and thus side effects due to an electrical leakage may be decreased.

### BRIEF DESCRIPTION OF THE DRAWINGS

FIGS. 1A to 1D are perspective views illustrating conventional implantable leads;
FIGS. 2A to 2C are side views illustrating an implantable hybrid lead according to an example embodiment of the present invention; and
FIG. 3 is a flow chart illustrating a method of manufacturing the implantable hybrid lead of FIGS 2A to 2C.

### * Reference numerals

110 : thin film electrode (line electrode)
120 : conduit having a fine channel
130 : electrode terminal

### DETAILED DESCRIPTION

The invention is described more fully hereinafter with Reference to the accompanying drawings, in which embodiments of the invention are shown. This invention may, however, be embodied in many different forms and should not be construed as limited to the embodiments set forth herein. Rather, these embodiments are provided so that this disclosure will be thorough and complete, and will fully convey the scope of the invention to those skilled in the art.

The terminology used herein is for the purpose of describing particular embodiments only and is not intended to be limiting of the invention.

As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

Hereinafter, the embodiments of the present invention will be described in detail with reference to the accompanied drawings. In addition, the same reference numerals will be used to refer to the same or like parts and any further repetitive explanation concerning the above elements will be omitted.

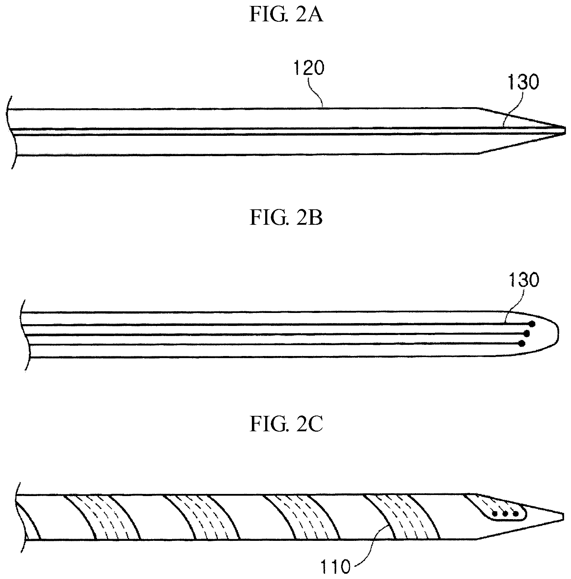

FIGS. 2A to 2C are side views illustrating an implantable hybrid lead according to an example embodiment of the present invention.

As illustrated in FIGS. 2A to 2C, the implantable hybrid lead according to the present example embodiment includes a conduit 120, a line electrode 110 and a plurality of electrode terminals 130. The conduit 120 has a fine channel through which a medicine is injected. The line electrode 110 is inserted to and is combined with an outside of the conduit 120, and applies electrical simulation to a selected portion of a living body. The electrode terminals 130 are disposed at an end of the conduit by a predetermined distance.

Here, the line electrode 110 is a thin film electrode or thin film multi electrodes array.

In addition, the line electrode 110 is coiled around an outer surface of the conduit 120 to be connected with the conduit 120, by a predetermined distance.

The conduit 120 is a flexible polydimethylsiloxane (PDMS) conduit.

The implantable hybrid lead may be used to be inserted into a human body in a short term or may be implanted into the human body for a long term. A thickness of the implantable hybrid lead may be between about 600 µm and about 2,000 µm, but not limited thereto.

In addition, the implantable hybrid lead is flexible so as to be inserted or positioned into an inside of a curved human body, and is biocompatible to be nontoxic with a long term usage.

A method of manufacturing the implantable hybrid lead is explained below.

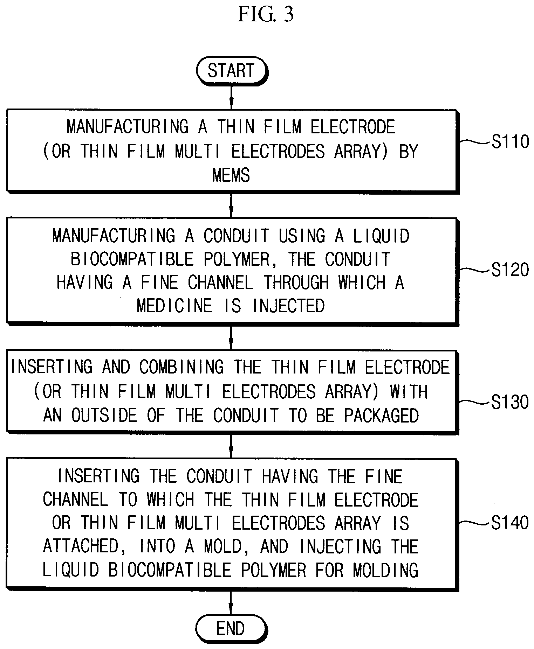

FIG. 3 is a flow chart illustrating a method of manufacturing the implantable hybrid lead of FIGS 2A to 2C.

Referring FIG. 3, in the method of manufacturing the implantable hybrid, a thin film electrode 110 or thin film multi electrodes array is manufactured by MEMS manufacturing processes (step S110). Here, the thin film electrode or the thin film multi electrodes array is formed on a substrate and then are removed from the substrate. For example, a UV photo-sensitive material such as polyimide having relatively high heat-resistance, chemical-resistance and biocompatibility is spin-coated on the substrate, to be formed as a bottom surface. Then, a metal thin film is formed on the bottom surface by chemical vapor deposition or physical vapor deposition, and is partially etched to form a predetermined pattern. Then, the UV photo-sensitive material is spin-coated on the predetermined pattern again, to be formed as an upper surface. Since the MEMS manufacturing processes are prior arts and further explanation on the processes are omitted. Here, the substrate may include a silicon wafer, a glass substrate, a quartz substrate, etc.

Then, the conduit 120 is manufactured using a liquid biocompatible polymer, and the conduit 120 has a fine channel through which a medicine is injected (step S120). Here, the conduit 120 may include the polydimethylsiloxane (PDMS) and is flexible. For example, a liquid polydimethylsiloxane is injected into a plastic or metal mold, and a heat is applied to the plastic or metal mold, so that the flexible conduit 120 may be manufactured by polymerization. Here, the thickness of the conduit 120 may be controlled by changing the thickness of the injected liquid polydimethylsiloxane.

Then, the thin film electrode 110 or the thin film multi electrodes array is inserted and combined with an outside of the conduit 120 to be packaged into together (step S130). Here, a surface of the outside of the conduit 120 is treated by an oxygen plasma treatment to increase a surface energy and then the package may be performed, but not limited thereto.

Then, the conduit 120 having the fine channel to which the thin film electrode 110 or the thin film multi electrodes array is attached, is inserted into a mold, and the liquid biocompatible polymer is injected for molding (step S140). For example, for manufacturing the molding, the PDMS conduit 120 to which the thin film electrode 110 or the thin film multi electrodes array is attached, is inserted into the metal or the plastic mold, and the liquid PDMS is injected.

Here, the thin film electrode 110 or the thin film multi electrodes array is coiled around the outer surface of the conduit 120 to be connected with the conduit 110, by a predetermined distance. For example, the thin film electrode 110 is coiled around the translucent PDMS conduit 120 like a ribbon, and is packaged by the molding. Here, the thin film electrode 110 is connected to the electrode terminal 130 of the end of the conduit 120.

In the present invention, the conduit, the PDMS conduit and the conduit having the fine channel are substantially same with each other.

Although the exemplary embodiments of the present invention have been described, it is understood that the present invention should not be limited to these exemplary embodiments but various changes and modifications can be made by one ordinary skilled in the art within the spirit and scope of the present invention as hereinafter claimed.

## Claims

1. An implantable hybrid lead comprising:
a conduit having a fine channel through which a medicine is injected;
a line electrode inserted to and combined with an outside of the conduit, and applying electrical simulation to a selected portion of a living body; and
a plurality of electrode terminals disposed at an end of the conduit by a predetermined distance.

2. The implantable hybrid lead of claim 1, wherein the line electrode is a thin film electrode or thin film multi electrodes array.

3. The implantable hybrid lead of claim 1, wherein the line electrode is coiled around an outer surface of the conduit to be connected with the conduit, by a predetermined distance.

4. A method of manufacturing an implantable hybrid lead, the method comprising:
manufacturing a thin film electrode by MEMS;
manufacturing a conduit using a liquid biocompatible polymer, the conduit having a fine channel through which a medicine is injected; and
inserting and combining the thin film electrode with an outside of the conduit to be packaged.

5. A method of manufacturing an implantable hybrid lead, the method comprising:
manufacturing thin film multi electrodes array by MEMS;
manufacturing a conduit using a liquid biocompatible polymer, the conduit having a fine channel through which a medicine is injected; and
inserting and combining the thin film multi electrodes array with an outside of the conduit to be packaged.

6. The method of one of claims 4 and 5, the method further comprising:
inserting the conduit having the fine channel to which the thin film electrode or the thin film multi electrodes array is attached, into a mold, and injecting the liquid biocompatible polymer for molding.

7. The method of one of claims 4 and 5, wherein the thin film electrode or the thin film multi electrodes array is coiled around an outer surface of the conduit to be connected with the conduit, by a predetermined distance.
